# EUROPÄISCHE PATENTSCHRIFT

(11) **EP 1 161 409 B1**
(45) Veröffentlichungstag und Bekanntmachung des Hinweises auf die Patenterteilung: **06.08.2003**
(21) Anmeldenummer: 00909260.2
(22) Anmeldetag: 28.02.2000
(51) Int. Cl.: C07C 45/65, C07C 49/597, C07C 49/603

(54) **VERFAHREN ZUR HERSTELLUNG CYCLISCHER ALPHA, BETA-UNGESÄTTIGTER KETONE**
METHOD OF PRODUCING CYCLIC, ALPHA, BETA-UNSATURATED KETONES
PROCEDE DE PRODUCTION DE CETONES CYCLIQUES ALPHA, BETA-INSATUREES

(30) Priorität: 12.03.1999 DE 19911169
(43) Veröffentlichungstag der Anmeldung: 12.12.2001
(73) Patentinhaber: BASF AKTIENGESELLSCHAFT, 67056 Ludwigshafen (DE)
(72) Erfinder: FISCHER, Rolf, D-69121 Heidelberg (DE); PINKOS, Rolf, D-67098 Bad Dürkheim (DE)
(86) Internationale Anmeldenummer: EP0001640
(87) Internationale Veröffentlichungsnummer: WO00055108

(56) Entgegenhaltungen:
- US-A- 3 364 264

## Beschreibung

Die vorliegende Erfindung betrifft ein Verfahren zur Herstellung von cyclischen, α,β-ungesättigten Ketonen durch Dehydrierung cyclischer Ketone bei erhöhter Temperatur in Abwesenheit von Sauerstoff oder weniger als 0,5 mol Sauerstoff pro mol Ausgangsketon in Gegenwart von Katalysatoren die eine BET-Oberfläche von mindestens 0,5 m²/g aufweisen.

Die Herstellung von cyclischen α,β-ungesättigten Ketonen aus den entsprechenden gesättigten Ketonen in der Gasphase ist z.B. für den Fall Cyclopentenon von F. Delles, J. Am. Chem. Soc. 91, 27 (1969) durch unkatalysierte Gasphasen-Pyrolyse bei 532 bis 581°C im Vakuum beschrieben. Allerdings sind die erzielbaren Cyclopenenon-Ausbeuten bzw. Selektivitäten sehr gering, da Zersetzungen vorherrschen.

Aus US 3 364 264 ist ferner die Umsetzung von Ketonen zu Enonen in der Gasphase an Oxidationskatalysatoren in Gegenwart von mindestens einem Mol Sauerstoff pro Mol Keton bekannt. Aus den Beispielen geht nicht hervor, welche Ausbeuten bzw. Selektivitäten erreicht werden, da Angaben über Gesamtumsatz oder Nebenprodukte fehlen. Unabhängig von den tatsächlich gegebenenfalls zu erzielenden Ausbeuten hat dieses Verfahren den grundsätzlichen Nachteil bei der Übertragung in den technischen Maßstab, daß besondere Sicherheitsmaßnahmen erforderlich sind, da man bei der Dosierung der großen Sauerstoffmengen in der Nähe oder innerhalb der Explosionsgrenzen arbeitet.

Es bestand daher die Aufgabe, ein Verfahren zu finden, mit dem man cyclische α,β-ungesättigter Ketone in technisch einfacher Weise in der Gasphase in hohen Ausbeuten kontinuierlich herstellen kann, ohne daß die Gefahr einer unkontrollierten Zersetzung besteht.

Diese Aufgabe wurde erfindungsgemäß gelöst mit einem Verfahren zur Herstellung, insbesondere der kontinuierlichen Herstellung von cyclischen, α,β-ungesättigten Ketonen der Formel II durch Dehydrierung von cyclischen Ketonen der Formel I 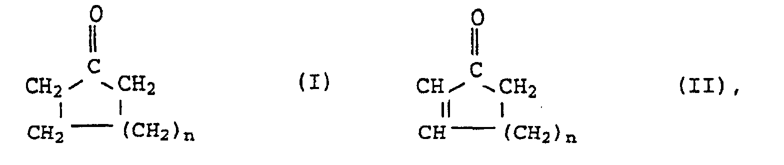 in denen n eine der Zahlen 1 bis 10 bedeutet und die gegebenenfalls substituiert sein können, bei erhöhter Temperatur in Gegenwart von Katalysatoren in der Gasphase, wobei man die Umsetzung in Abwesenheit von Sauerstoff oder in Abwesenheit von weniger als 0,5 mol Sauerstoff/mol der Verbindung I bei Temperaturen von 250 bis 600°C und mit Katalysatoren mit einer BET-Oberfläche größer 0,5 m²/g durchführt.

Die cyclischen Enone bzw. Ketone der Formel II und I können an den Ring-C-Atomen noch Substituenten tragen. Solche Substituenten sind z.B. Alkyl- oder Arylreste. Bevorzugte Ausgangs-Ketone sind Cyclohexanon und insbesondere Cyclopentanon.

Die Umsetzung wird bevorzugt in Abwesenheit von Sauerstoff durchgeführt, jedoch kann die Umsetzung auch mit gewissen Mengen Sauerstoff, z.B. mit bis zu 0,2 mol Sauerstoff pro mol Keton durchgeführt werden.

Die Reaktionstemperaturen des erfindungsgemäßen Verfahrens liegen im allgemeinen zwischen 250 und 600°C, bevorzugt bei 300 bis 575°C und insbesondere bei 350 bis 550°C. Der Reaktionsdruck beträgt im allgemeinen zwischen 0,1 und 10 bar, bevorzugt 0,5 bis 6 bar und insbesondere 0,9 bis 3 bar.

Die Umsetzung der cyclischen Ketone kann mit oder ohne Trägergas erfolgen. Sofern mit Trägergas gearbeitet wird, werden inerte Gase wie z.B. Methan, Stickstoff oder Argon verwendet.

Soll Sauerstoff zudosiert werde, so wird dies entweder durch Dosierung von Luft oder von Mischungen von Luft mit Inertgas oder mit reinem Sauerstoff bzw. Mischungen von Sauerstoff mit Inertgasen vorgenommen. Dabei sollte das Mol-Verhältnis Sauerstoff zu Keton in der Regel 0,1 nicht übersteigen. Bevorzugt liegt das Molverhältnis unter 0,01, besonders bevorzugt wird in Abwesenheit von Sauerstoff gearbeitet.

Das erfindungsgemäße Verfahren kann in Gegenwart von Wasser durchgeführt werden. Die Wassermenge ist im allgemeinen unkritisch, liegt aber bevorzugt zwischen 0,01 bis 1 kg, besonders bevorzugt zwischen 0,05 bis 0,2 kg Wasser pro kg cyclischem Keton. Der Zusatz Wasser bewirkt im allgemeinen eine Verlängerung der Katalysatorstandzeit.

Als Katalysatoren eignen sich prinzipiell alle Feststoffe, die sich unter den Reaktionsbedingungen nicht verändern, mit einer BET-Oberfläche über 0,5 m²/g. Bevorzugte Katalysatoren haben Oberflächen über 1 m²/g. Nach oben sind der BET-Oberfläche prinzipiell keine Grenzen gesetzt, doch wird aus praktischen Erwägungen die BET-Oberfläche in der Regel 2000 m²/g nicht überschreiten. Demgemäß beträgt die BET-Oberfläche der erfindungsgemäß zu verwendenden Katalysatoren im allgemeinen 0,5 bis 2000, bevorzugt 1 bis 500 und insbesondere 2 bis 200 m²/g.

Als Katalysatormassen kommen insbesondere oxidische Materialien in Betracht. Dies sind vor allem Katalysatoren, die Oxide der Elemente der Gruppen 2 bis 14 des Periodensystems der Elemente enthalten oder aus diesen bestehen.

Beispiele hierfür sind Magnesiumoxid, Titandioxid, Zirkondioxid, Chrom-III-oxid, Kobaltoxid, Kupferoxid, Zinkoxid, Aluminiumoxid und Siliciumdioxid.

Die Oxide können einheitlich oder gemischt sein. Verschiedene Oxide können durch gemeinsame Fällung homogene Mischoxide bilden oder in mechanischer Mischung vorliegen.

Weiterhin sind als katalytisch wirkende Grundmaterialien mit großer Oberfläche Aktivkohle oder Siliciumnitrid zu nennen.

Die vorgenannten katalytischen Materialien mit großer Oberfläche können noch, z.B. in Mengen von 0,01 bis 5 Gew.-%, Verbindungen aus Elementen der 1., 15. und 16. Gruppe des Periodensystems der Elemente enthalten. Diese Elemente sind z.B. Na, K, P oder S, in der Regel in Form der Oxide.

Schließlich können, vor allem auf die genannten oxidischen Grundmaterialien noch metallische Komponenten, bevorzugt ebenfalls in Form der Oxide, der 7. bis 11. Gruppe des Periodensystems der Elemente aufgebracht sein. Beispielsweise sind dafür Rhenium, Ruthenium, Kobalt, Rhodium, Nickel, Palladium, Platin, Kupfer und Silber zu nennen.

Im einzelnen kommen dabei z.B. folgende Katalysatoren in Betracht: CuO, AgO, PdO, NiO, Mn₂O₃ oder Re₂O₇
auf ZnO, CaO, BaO, SiO₂ oder Al₂O₃. Die Herstellung dieser Katalysatoren erfolgt in an sich bekannter Weise, z.B. durch Tränken und Calcinieren oder durch Fällen von Hydroxiden oder Carbonaten und Calcinieren, wie dies z.B. in Charles N. Satterfield, Heterogeneous Catalysis in Industrial Practice, 2. Aufl., New York Mc Graw-Hill, beschrieben ist.

Das erfindungsgemäße Verfahren kann über fest angeordneten oder bewegten Katalysatoren durchgeführt werden. Beispiele für geeignete Reaktortypen sind Festbett-Reaktoren, Wirbelbett-Reaktoren und Riser-Reaktoren.

Sofern die Katalysatoraktivität, z.B. durch organische Ablagerung, nachläßt, kann im allgemeinen der Katalysator auf anorganischer Basis durch Abbrennen der Ablagerungen vollständig regeneriert werden, z.B. bei 400 bis 500°C mit Luft.

Die Umsetzung kann in geradem Durchgang erfolgen, wobei das entstehende Produktgemisch anschließend aufgearbeitet wird und gegebenenfalls nicht umgesetzter Edukt wieder zurückgeführt wird, oder das Reaktionsprodukt kann vor Aufarbeitung nochmals in den Reaktor zurückgeführt werden, um den Umsatz zu erhöhen.

Cyclische Enone wie z.B. Cyclopentenon oder Cyclohexenon sind gesuchte Zwischenverbindungen zur Herstellung von Pharmawirkstoffen oder Pflanzenschutzmitteln.

Das erfindungsgemäße Verfahren wird durch die nachfolgenden Beispiele näher erläutert, aber nicht eingeschränkt. Die %-Angaben in den Beispielen wurden durch gaschromatographische Analyse ermittelt.

### Beispiel 1

In ein Quarzrohr mit außenliegender Elektroheizung wurden ca. 100 ml ZnO (BET-Oberfläche 15 m²/g; 3 x 5 mm Stränge) und über den Katalysatorsträngen ca. 100 ml Glasringe als Verdampfungszone gefüllt. Der Reaktor wurde mittels Elektroheizung auf die gewünschte Reaktionstemperatur gebracht und es wurde ein Gasstrom von 20 NL/Stunde Stickstoff eingestellt. Danach wurden ca. 20 ml/Stunde Cyclopentanon in Rieselfahrweise eingetragen. Der gasförmige Reaktoraustrag wurde mittels einer Wasserkühlung kondensiert, das aufgefangene Produkt anschließend gaschromatographisch analysiert. In Tab. 1 sind die Reaktionstemperaturen sowie die dabei erhaltenen Reaktoraustragszusammensetzungen (Gew.-%) wiedergegeben.

### Beispiele 2-7

Man verfuhr wie in Beispiel 1 beschrieben unter Verwendung der in Tabelle 1 angegebenen weiteren Katalysatoren, und erhielt die dort wiedergegebenen Ergebnisse. In Beispiel 5 wurden zusätzlich zu 20 NL/h Stickstoff noch 4 NL/h Luft zudosiert. Die in Beispiel 5 verwendeten Cu-Ringe wurden vor der Reaktion bei 500°C mit Sauerstoff behandelt. In Beispiel 7 wurde Cyclopentanon eingesetzt, das 5 % Wasser enthielt.

**Tabelle 1**

| Beispiel | Katalysator | Temperatur | Cyclopentanon | Cyclopentenon |
|---|---|---|---|---|
| 1 | ZnO | 400 | 42 | 7,1 |
| | BET-Oberfläche | 450 | 77 | 16 |
| | 15 m²/g | 500 | 67 | 29 |
| 2 | CuO(20%)/ZnO | 350 | 66 | 6,2 |
| | (80%) | 400 | 61 | 25,3 |
| | BET-Oberfläche | 450 | 66 | 26,9 |
| | 15 m²/g | 500 | 57 | 32,5 |
| 3 | CuO(17,5%)/SiO₂ | 350 | 70 | 4,8 |
| | BET-Oberfläche | 400 | 68 | 10,4 |
| | 29 m²/g | 450 | 66 | 21 |
| | | 500 | 63 | 26 |
| 4 | CaO(44%)/ZnO(56%) | 400 | 76 | 11 |
| | BET-Oberfläche | 450 | 68 | 28 |
| | 15 m²/g | 500 | 56 | 39 |
| 5 | Cu-Ringe | 450 | 96,7 | 2,7 |
| | BET-Oberfläche | 500 | 92,2 | 6,6 |
| | 1 m²/g | | | |
| 6 | CuO | 400 | 90,4 | 3,5 |
| | (10%)/Aktivkohle | 450 | 83,4 | 7,6 |
| | BET-Oberfläche | 500 | 72,8 | 18,9 |
| | 1000 m²/g | | | |
| 7 | ZnO | 450 | 89 | 10,5 |
| | BET-Oberfläche | | | |
| | 15 m²/g | | | |
| 8 | Pd (9,5%)/Pt | 400 | 79 | 8,5 |
| | (0,5%)/ZrO₂ | | | |
| | BET-Overfläche | | | |
| | 70 m²/g | | | |
| 9 | Fe₂O₃(77%)/K₂O | 400 | 93,7 | 3,1 |
| | (12.5%)/WO₃ | | | |
| | (3,8%)/Ce₂O₃ | | | |
| | (4,7%)/CaO(2%) | | | |
| | BET-Oberfläche | | | |
| | 3,5 m²/g | | | |

### Beispiel 10

Analog Beispiel 1 wurde an 100 ml ZnO Cyclopentanon bei 500°C umgesetzt. Nach ca. 48 h Laufzeit und einer Zulaufmenge von ca. 1 Liter wurden im Austrag 85,1 Gew.-% Cyclopentanon und 14,5 Gew.-% Cyclopentenon gefunden. Die gesammelten Austräge wurden bei 1013 mbar an einer 40 cm Füllkörperkolonne aufdestilliert. Es wurde Cyclopentenon mit einer Reinheit von ca. 99,6 % erhalten.

### Beispiel 11

Wie in Beispiel 1 beschrieben, wurde Cyclopentanon an ZnO bei 400°C umgesetzt. Nach einer Einfahrphase von 0,5 h wurde Reaktionsprodukt über ca. 20 h gesammelt. Es hatte folgende Zusammensetzung: Cyclopentanon 95,1 Gew.-%, Cyclopentenon 4,2 Gew.-%. Anschließend wurde dieses Produktgemisch nochmals eingesetzt. Im Reaktionsaustrag fanden sich 92,5 % Gew.-% Cyclopentanon und 6,6 Gew.-% Cyclopentenon.

### Beispiel 12

Entsprechend den Angaben des Beispiels 2 (CuO/ZnO; BET-Oberfläche 15 m²/g) wurde Cycloheptanon bei 400°C umgesetzt. Im Austrag wurden 95 Gew.-% nicht umgesetztes Edukt, 1 Gew.-% Cycloheptenon und 1,3 Gew.-% Kresol sowie weitere mengenmäßig unbedeutende Produkte gefunden.

## Patentansprüche

1. Verfahren zur Herstellung von cyclischen, α,β-ungesättigten Ketonen der Formel II durch Dehydrierung von cyclischen Ketonen der Formel I 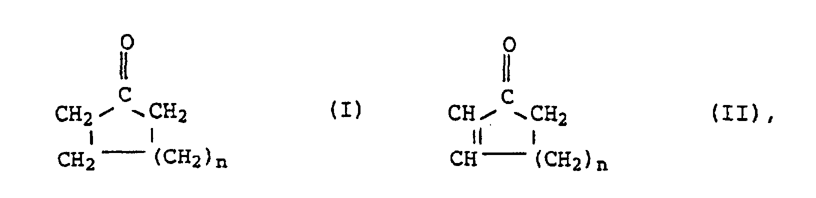 in denen n eine der Zahlen 1 bis 10 bedeutet und die gegebenenfalls substituiert sein können, bei erhöhter Temperatur in Gegenwart von Katalysatoren in der Gasphase, **dadurch gekennzeichnet, daß** man die Umsetzung in Abwesenheit von Sauerstoff oder in Anwesenheit von weniger als 0,5 mol Sauerstoff/mol der Verbindung I bei Temperaturen von 250 bis 600°C und mit Katalysatoren mit einer BET-Oberfläche größer 0,5 m²/g durchführt.

2. Verfahren gemäß Anspruch 1, **dadurch gekennzeichnet, daß** man die Umsetzung in Abwesenheit von Sauerstoff oder in Gegenwart von weniger als 0,2 mol Sauerstoff/mol der Verbindung der Formel I durchführt.

3. Verfahren gemäß Anspruch 1, **dadurch gekennzeichnet, daß** man die Umsetzung in Abwesenheit von Sauerstoff oder in Gegenwart von weniger als 0,1 mol Sauerstoff/mol der Verbindung der Formel I durchführt.

4. Verfahren gemäß Anspruch 1, **dadurch gekennzeichnet, daß** man die Umsetzung bei Drücken zwischen 0,1 und 10 bar durchführt.

5. Verfahren gemäß Anspruch 1, **dadurch gekennzeichnet, daß** man die Umsetzung in Gegenwart von Wasser durchführt.

6. Verfahren gemäß Anspruch 1, **dadurch gekennzeichnet, daß** man die Umsetzung in Gegenwart oxidischer Katalysatoren durchführt.

7. Verfahren gemäß Anspruch 1, **dadurch gekennzeichnet, daß** man die Umsetzung in Gegenwart von Katalysatoren durchführt, die Oxide der 2. bis 14. Gruppe des Periodensystems der Elemente enthalten oder aus diesen bestehen.

8. Verfahren gemäß Anspruch 7, **dadurch gekennzeichnet, daß** man die Umsetzung in Gegenwart von Katalysatoren durchführt, die zusätzlich Elemente der 7. bis 12. Gruppe des Periodensystems der Elemente oder deren Oxide aufgebracht enthalten.

9. Verfahren gemäß Anspruch 1, **dadurch gekennzeichnet, daß** man als cyclisches Ausgangsketon der Formel I Cyclopentanon verwendet.

10. Verfahren gemäß Anspruch 1, **dadurch gekennzeichnet, daß** man als cyclisches Ausgangsketon der Formel I Cyclohexanon verwendet.

## Claims

1. A process for the preparation of cyclic, α,β-unsaturated ketones of formula II by dehydrogenation of cyclic ketones of formula I 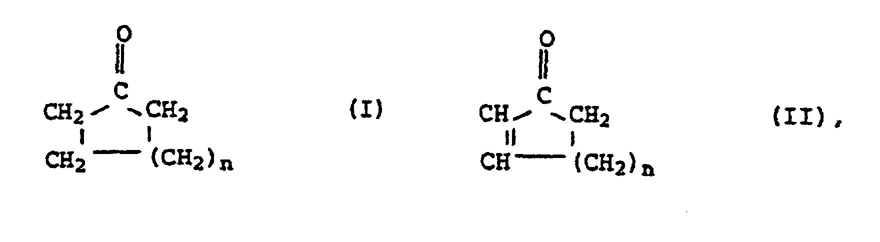 in which n denotes an integer from 1 to 10, and which may be substituted, at elevated temperature in the presence of a catalyst in the gas phase, **characterized in that** the reaction is carried out in the absence of oxygen or in the presence of less than 0.5 mol of oxygen/mol of compound I at a temperature ranging from 250° to 600°C and using a catalyst having a BET surface area of more than 0.5 m²/g.

2. A process as defined in claim 1, **characterized in that** the reaction is carried out in the absence of oxygen or in the presence of less than 0.2 mol of oxygen/mol of compound of formula I.

3. A process as defined in claim 1, **characterized in that** the reaction is carried out in the absence of oxygen or in the presence of less than 0.1 mol of oxygen/mol of compound of formula I.

4. A process as defined in claim 1, **characterized in that** the reaction is carried out under a pressure of from 0.1 to 10 bar.

5. A process as defined in claim 1, **characterized in that** the reaction is carried out in the presence of water.

6. A process as defined in claim 1, **characterized in that** the reaction is carried out in the presence of an oxidic catalyst.

7. A process as defined in claim 1, **characterized in that** the reaction is carried out in the presence of a catalyst containing or consisting of Group 2 to Group 14 oxides.

8. A process as defined in claim 2, **characterized in that** the reaction is carried out in the presence of a catalyst to which there has been additionally applied a Group 7 to Group 12 element or an oxide thereof.

9. A process as defined in claim 1, **characterized in that** the cyclic starting ketone of formula I used is cyclopentanone.

10. A process as defined in claim 1, **characterized in that** the cyclic starting ketone of formula I used is cyclohexanone.

## Revendications

1. Procédé pour la préparation de cétones cycliques insaturées en positions α,β de formule II, au moyen d'une déshydrogénation de cétones cycliques de formule I 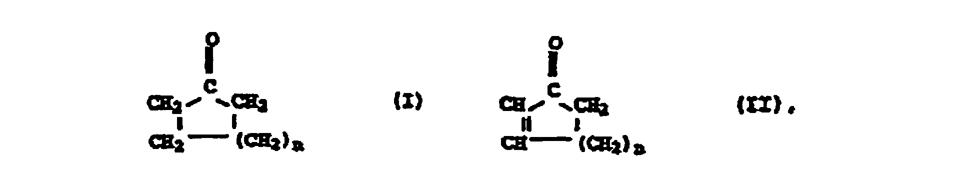 formules dans lesquelles n représente un nombre allant de 1 à 10, et cétones qui sont éventuellement substituées, à une température élevée, en présence de catalyseurs, en phase gazeuse, **caractérisé en ce que** l'on réalise la réaction en l'absence d'oxygène ou en présence de moins de 0,5 mole d'oxygène/mole du composé I, à des températures allant de 250°C à 600°C et en mettant en oeuvre des catalyseurs présentant une surface spécifique BET supérieure à 0,5 m²/g.

2. Procédé selon la revendication 1, **caractérisé en ce que** l'on réalise la réaction en l'absence d'oxygène ou en présence de moins de 0,2 mole d'oxygène/mole du composé de formule I.

3. Procédé selon la revendication 1, **caractérisé en ce que** l'on réalise la réaction en l'absence d'oxygène ou en présence de moins de 0,1 mole d'oxygène/mole du composé de formule I.

4. Procédé selon la revendication 1, **caractérisé en ce que** l'on réalise la réaction à des pressions allant de 0,1 bar à 10 bars.

5. Procédé selon la revendication 1, **caractérisé en ce que** l'on réalise la réaction en présence d'eau.

6. Procédé selon la revendication 1, **caractérisé en ce que** l'on réalise la réaction en présence de catalyseurs d'oxyde.

7. Procédé selon la revendication 1, **caractérisé en ce que** l'on réalise la réaction en présence de catalyseurs qui contiennent ou qui sont composés d'oxydes du 2^{ème} au 14^{ème} groupe de la Classification Périodique des Eléments.

8. Procédé selon la revendication 7, **caractérisé en ce que** l'on réalise la réaction en présence de catalyseurs qui contiennent, en plus et sous forme supportée, des éléments du 7^{ème} au 12^{ème} groupe de la Classification Périodique des Eléments, ou leurs oxydes.

9. Procédé selon la revendication 1, **caractérisé en ce que** l'on met en oeuvre, en tant que cétone cyclique de départ de formule I, la cyclopentanone.

10. Procédé selon la revendication 1, **caractérisé en ce que** l'on met en oeuvre, en tant que cétone cyclique de départ de formule I, la cyclohexanone.
